(19) Europäisches Patentamt
European Patent Office
Office européen des brevets

(11) Veröffentlichungsnummer: **0 126 887**
**B1**

(12) # EUROPÄISCHE PATENTSCHRIFT

(45) Veröffentlichungstag der Patentschrift: **04.06.86**

(51) Int. Cl.[4]: **C 07 C 99/12**, C 07 C 101/04, C 12 P 13/06

(21) Anmeldenummer: **84103330.1**

(22) Anmeldetag: **27.03.84**

(54) **Verfahren zur Gewinnung von reinem L-Leucin.**

(30) Priorität: **25.05.83 DE 3318932**

(43) Veröffentlichungstag der Anmeldung: **05.12.84 Patentblatt 84/49**

(45) Bekanntmachung des Hinweises auf die Patenterteilung: **04.06.86 Patentblatt 86/23**

(84) Benannte Vertragsstaaten: **AT BE CH FR GB LI NL SE**

(56) Entgegenhaltungen:
**CH - A - 284 412**

**LIEBIGS ANNALEN DER CHEMIE, Heft 11, 15 November 1983, Seiten 2052-2054, Verlag Chemie, WEINHEIM, (DE) J. MARTENS et al.: "Enzymatic separation of 1-leucine and 1-isoleucine"**

(73) Patentinhaber: **Degussa Aktiengesellschaft, Weissfrauenstrasse 9, D-6000 Frankfurt am Main 1 (DE)**

(72) Erfinder: **Kleemann, Axel, Dr. Dipl.-Chem., Greifenhagenstrasse 25, D-6450 Hanau 9 (DE)**
Erfinder: **Martens, Jürgen, Dr. Dipl.-Chem., Hochstrasse 10, D-8755 Alzenau (DE)**
Erfinder: **Weigel, Horst, Odenwaldstrasse 56, D-6458 Rodenbach (DE)**

EP 0 126 887 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Gewinnung von reinem L-Leucin aus Aminosäuregemischen, die mindestens 45 Gewichtsprozent L-Leucin, höchstens 40 Gewichtsprozent L-Isoleucin und höchstens 25 Gewichtsprozent an anderen Aminosäuren, jeweils bezogen auf trockensubstanz, enthalten.

L-Leucin ist als Pharmawirkstoff z.B. Bestandteil von Infusionslösungen auf Aminosäurebasis. Bis heute erfolgt die industrielle Gewinnung von L-Leucin nach dem sogenannten Extraktionsverfahren. Dazu werden Proteine zu Aminosäuregemischen hydrolysiert und aus diesen wird durch fraktionierte Kristallisation und/oder chromatographische Verfahren (Ionenaustausch, Ionenausschluss und/oder Molekularsiebeffekt) ein technisches L-Leucin gewonnen. Das so gewonnene technische L-Leucin enthält neben anorganischen Salzen, wie Natriumchlorid, Natriumsulfat, Ammoniumchlorid oder Ammoniumsulfat, noch andere Aminosäuren, insbesondere L-Isoleucin. Zur Herstellung von L-Leucin in Pharmaqualität müssen diese Verunreinigungen entfernt werden, wobei insbesondere die Abtrennung des L-Isoleucins Schwierigkeiten bereitet, da das Isoleucin die gleiche Summenformel $C_6H_{13}NO_2$ wie Leucin hat und sich diese beiden Aminosäuren nur durch die Struktur der aliphatischen Seitenkette R unterscheiden:

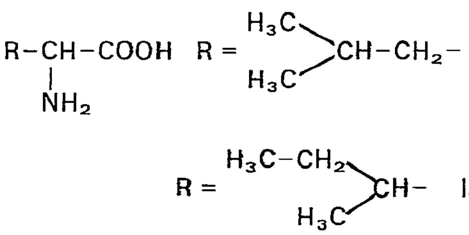

Leucin

Isoleucin

Bedingt durch diese sehr ähnlichen Strukturmerkmale zeigen diese Aminosäuren im physikalischen und chemischen Verhalten sehr ähnliche Eigenschaften. Dies ist auch der Grund dafür, dass kommerzielles L-Leucin häufig nicht unbeträchtliche Mengen an L-Isoleucin neben anderen Aminosäuren (z.B. L-Valin, manchmal auch L-Methionin) enthält. Auf diesen Umstand weist auch Richard J. Block [Arch. Biochem. *11*, 501 (1946), dort Seite 512] hin.

Die schwierige Aufreinigung von technischem L-Leucin wurde bereits über Kupferkomplexe realisiert. In ähnlichen Verfahren wurden auch die Kobaltkomplexe der Aminosäuren durch Extraktion mit Alkohol aufgetrennt. Bei diesen Verfahren ergibt sich jedoch das Problem der Rückgewinnung der Metalle und der weiteren Reinigung des L-Leucins.

Von anderen Autoren wurde die Fällung von Leucin mit aromatischen Sulfonsäuren beschrieben. So wird die Verwendung von 2-Bromtoluol-5-sulfonsäure oder Naphthalin-2-sulfonsäure für die Fällung von L-Leucin vorgeschlagen. Auch Benzolsulfonsäure sowie p-Toluolsulfonsäure wurden für die L-Leucin-Gewinnung, ausgehend von technischem L-Leucin, eingesetzt. Bei diesen Verfahren müssen die Präzipitate durch zahlreiche Umkristallisationen gereinigt werden und ein besonderes Problem bereitet die Abtrennung der oft stark giftigen Fällungsmittel.

Weiterhin wurden an L-Leucin reiche Fraktionen aus sauren Proteinhydrolysaten dadurch aufgereinigt, dass man bei einem bestimmten pH-Wert unter Zusatz von Wasser auf eine L-Isoleucinkonzentration von ca. 1,5% einstellte und nach Ermittlung des Methionin-Gehaltes durch Zusatz von Wasserstoffperoxid das Methionin oxidierte. Es folgte eine Aktivkohleklärung, Einstellen des pH-Wertes auf 1,0 bis 1,5, Abkühlen und Abtrennen eines rohen L-Leucins. Dieses wurde erneut bei pH 0,5 gelöst und durch Fällung bei pH 1,0 bis 2,0 weiter gereinigt. Dieses Verfahren wird so oft wiederholt, bis die gewünschte Reinheit des L-Leucins erreicht ist. Bei diesem Verfahren, das in der Europäischen Patentschrift 14867 beschrieben ist, muss man sehr verdünnt arbeiten und ausserdem ist der Prozess derart vielstufig, dass eine Anwendung im industriellen Massstab kaum in Frage kommt.

Weiterhin haben die bekannten Verfahren der Aufreinigung von technischem L-Leucin zur Gewinnung von reinem L-Leucin in Pharmaqualität einen entscheidenden Mangel: Über die Enantiomerenreinheit des L-Leucins wird nichts gesagt. Leucin gehört aber zu den unter den Bedingungen der üblichen sauren Proteinhydrolyse am schnellsten racemisierenden Aminosäuren [Liebigs Ann. Chem. *1981*, 354 bis 365]. Die unterschiedliche biologische Wirksamkeit von D-Leucin und L-Leucin ist bekannt, so dass ein Verfahren, das gleichzeitig die Abtrennung von D-Leucin und von sonstigen Verunreinigungen aus technischem L-Leucin erlaubt, sehr erwünscht ist.

Das erfindungsgemässe Verfahren ist nun dadurch gekennzeichnet, dass man

a) das Aminosäuregemisch in an sich bekannter Weise acetyliert,

b) aus dem rohen Gemisch der Acetylierungsprodukte durch Ansäuern mit einer Mineralsäure ein an N-Acetyl-L-leucin angereichertes Gemisch an N-Acetylaminosäuren ausfällt,

c) dieses an N-Acetyl-L-leucin angereicherte Gemisch in wässeriger Lösung mit einer Konzentration zwischen 0,1 und 1,5 Mol/l N-Acetyl-L-leucin bei einem pH zwischen 6 und 8 und einer Temperatur zwischen 10 und 40° C in Gegenwart eines Effektors einer Verseifung durch eine L-Aminosäureacylase unterwirft, bis 30 bis 95% des eingesetzten N-Acetyl-L-leucins zur freien Aminosäure verseift sind, und

d) aus dem rohen Verseifungsgemisch das L-Leucin isoliert.

Bei den beim erfindungsgemässen Verfahren einzusetzenden Aminosäuregemischen handelt es sich im allgemeinen um technisches L-Leucin in Form einer bereits an L-Leucin angereicherten Fraktion aus einem proteinhydrolysat. Besonders geeignet sind Aminosäuregemische, die bezogen auf Trockensubstanz, 45 bis 90 Gewichtsprozent L-Leucin enthalten. Ein besonders geeignetes

technisches L-Leucin stammt aus der Fraktionierung von Blutmehlhydrolysaten und enthält 70 bis 80 Gewichtsprozent L-Leucin, 5 bis 15 Gewichtsprozent L-Isoleucin und maximal 25 Gewichtsprozent andere Aminosäuren.

Das Aminosäuregemisch wird zunächst in an sich bekannter Weise acetyliert. Die Acetylierung kann mit Acetylchlorid oder Acetanhydrid oder auch mit Keten nach dem aus der DE-OS 27 41 081 bekannten Verfahren vorgenommen werden.

Anschliessend wird aus dem rohen Gemisch der Acetylierungsprodukte durch Ansäuern mit einer Mineralsäure, z.B. Salzsäure oder Schwefelsäure, ein Gemisch von N-Acetylaminosäuren ausgefällt. Zweckmässigerweise wird dabei auf einen pH zwischen 0,5 und 2 angesäuert. Dabei findet bereits eine Anreicherung von N-Acetyl-L-leucin statt. Der etwaige Gehalt an anderen N-Acetylaminosäuren als N-Acetyl-L-leucin und N-Acetyl-L-isoleucin nimmt ab. Durch eine anschliessende Umkristallisation aus Wasser, einem mit Wasser mischbaren aliphatischen Alkohol mit 1 bis 4 Kohlenstoffatomen, wie Methanol, Ethanol, n-Propanol, Isopropylalkohol, n-Butanol, Isobutylalkohol oder tert. Butylalkohol, oder einem Gemisch von Wasser und einem solchen Alkohol kann der Gehalt an sonstigen N-Acetylaminosäuren auf normalerweise weniger als 4, in vielen Fällen weniger als 1 Gewichtsprozent, bezogen auf Trockensubstanz, vermindert werden.

Das an N-Acetyl-L-leucin angereicherte Gemisch, das nach einer etwaigen Umkristallisation fast nur noch mit N-Acetyl-L-isoleucin verunreinigt ist, wird nun der Verseifung durch eine L-Aminosäureacylase unterworfen. Die Einstellung des pH auf den Bereich zwischen 6 und 8 kann beispielsweise durch Ammoniak, vorzugsweise aber durch Natronlauge erfolgen. Als Effektoren können die üblicherweise bei der Racematspaltung von N-Acetyl-DL-α-aminocarbonsäuren mittels einer L-Aminosäureacylase zugesetzten dienen, beispielsweise die Ionen $Ca^{2+}$, $Fe^{2+}$, $Mn^{2+}$, $Mg^{2+}$, $Zn^{2+}$ und vorzugsweise $Co^{2+}$. Sie werden zweckmässigerweise in einer Konzentration zwischen $1 \cdot 10^{-5}$ und $1 \cdot 10^{-1}$ Mol/l und beispielsweise in Form der entsprechenden Cloride zugesetzt. In vielen Fällen ist es vorteilhaft, dem Reaktionsgemisch zusätzlich geringe Mengen eines Biocids, beispielsweise p-Hydroxybenzoesäure-n-propylester, zuzusetzen.

Als L-Aminosäureacylase wird bevorzugt eine Nierenacylase eingesetzt. Sie kann entweder in der handelsüblichen nativen Form angewandt werden oder als Immobilisat auf organischen Trägermaterialien, wie vernetzter Agarose, Dextrangelen, Cellulose, Hydroxyethylcellulose oder Mischpolymerisaten von Acrylamid mit vernetzenden Comonomeren, oder auf anorganischen Trägermaterialien, wie verschiedenen porösen oxidischen Materialien oder insbesondere Glaskügelchen.

Bei Anwendung einer relativ grossen Menge an L-Aminosäureacylase kann die für die Verseifung erforderliche Reaktionszeit verkürzt werden. Nimmt man längere Reaktionszeiten in Kauf, kann die Einsatzmenge an L-Aminosäureacylase erheblich vermindert werden. Zwischen der Reaktionszeit und der Einsatzmenge an L-Aminosäureacylase besteht demnach eine ausgeprägte gegenseitige Abhängigkeit.

Die Verseifung, also die Reaktionsstufe c), wird dann abgebrochen, wenn 30 bis 95%, vorzugsweise 45 bis 85%, insbesondere 50 bis 80%, des ursprünglich vorhandenen N-Acetyl-L-leucins zum freien L-Leucin verseift sind. Die Verseifungsreaktion wird analytisch verfolgt, z.B. mittels Hochdruckflüssigkeitschromatographie oder mit einem Aminosäureanalysator.

Die Verseifungsreaktion kann diskontinuierlich oder kontinuierlich durchgeführt werden. Für die diskontinuierliche Reaktionsführung arbeitet man in einem Rührkessel oder in einem Tank mit Umlauf. Eine kontinuierliche Reaktionsführung kann beispielsweise mittels eines Enzym-Membranreaktors realisiert werden.

Weist das für die Verseifungsreaktion eingesetzte Gemisch eine relativ hohe Konzentration an N-Acetyl-L-leucin auf, beispielsweise zwischen 1,0 und 1,5 Mol/l, so beginnt schon nach kurzer Zeit ein farbloser Niederschlag auszukristallisieren, der aus reinem L-Leucin besteht. Wird das Gemisch in niedrigeren Konzentrationen eingesetzt, ist es zweckmässig, das rohe Verseifungsgemisch unter schonenden Bedingungen aufzukonzentrieren, wobei dann ebenfalls reines L-Leucin auskristallisiert. In beiden Fällen wird das reine L-Leucin durch Abfiltrieren oder Abzentrifugieren isoliert, gegebenenfalls nach vorherigem Klassifizieren zwecks Abtrennung einer etwa eingesetzten immobilisierten L-Aminosäureacylase.

Wird die Verseifung mit nativer L-Aminosäureacylase vorgenommen, so kann diese nach der Abtrennung des L-Leucins durch Ultrafiltration, z.B. an Hohlfasermembranen, zurückgewonnen und erneut eingesetzt werden.

Unter Umständen kann es vorteilhaft sein, das L-Leucin in mehreren Fraktionen zu isolieren, d.h. nach Abtrennung einer ersten Fraktion die Verseifung fortzusetzen und weitere Fraktionen von L-Leucin zu gewinnen. Eine gegebenenfalls wünschenswerte weitere Reinigung der einzelnen Fraktionen oder der Gesamtmenge an abgetrenntem L-Leucin kann durch Umkristallisation aus Wasser erfolgen.

In der nach Abtrennung des L-Leucins und gegebenenfalls der L-Aminosäureacylase verbleibenden Lösung ist das N-Acetyl-L-isoleucin merklich angereichert. Diese Lösung kann, zweckmässigerweise nach Bestimmung ihrer Zusammensetzung durch Hochdruckflüssigkeitschromatographie oder mit einem Aminosäureanalysator, entweder in die Acetylierungsstufe zurückgeführt oder auf L-Isoleucin aufgearbeitet werden.

Die Erfindung wird durch die nachfolgenden Beispiele näher erläutert. Für diese Beispiele gilt folgendes:

Die Aktivität der L-Aminosäureacylase wird in units (U) angegeben. 1 U verseift bei pH 7,0 und 25° C 1 μMol N-Acetyl-L-methionin pro Stunde.

Die für das L-Leucin angegebenen Drehwerte

$[\alpha]_D^{25}$ werden bei c = 4 in 6N Salzsäure gemessen. Der Drehwert von reinem L-Leucin in Pharmaqualität beträgt laut US-Pharmacopoe XX unter diesen Bedingungen

$$[\alpha]_D^{25} = +14{,}9° \text{ bis } +17{,}3°.$$

Die Bestimmung der Zusammensetzung der eingesetzten Aminosäuregemische erfolgt in einem Aminosäureanalysator.

Die Bestimmung der Zusammensetzung der zur enzymatischen Verseifung eingesetzten Gemische von N-Acetylaminosäuren erfolgt über die Drehwerte.

Prozentangaben bedeuten, sofern nicht anders angegeben, Gewichtsprozente.

*Beispiel 1:*

Ein als Nebenprodukt der Sojabohnenhydrolyse angefallenes technisches Leucin folgender relativer Aminosäurezusammensetzung:

| | |
|---|---|
| Ile | 12,4% |
| Leu | 83,2% |
| Val | 0,9% |
| Met | 2,4% |
| Ala | 0,5% |
| Ser | 0,2% |
| andere Aminosäuren zusammengenommen | 0,4% |

wurde acetyliert. Dazu wurden 525 g dieser Mischung in 2 Litern 2 N Natronlauge gelöst und bei 0° C innerhalb einer Stunde gleichzeitig tropfenweise mit 450 ml Acetanhydrid und 850 ml 5 N Natronlauge versetzt, wobei darauf geachtet wurde, dass der pH-Wert in einem Bereich zwischen pH 10 und 12 blieb. Nach beendeter Zugabe wurde noch eine Stunde bei +5° C gerührt. Die braune Lösung wurde mit konzentrierter Salzsäure verrührt, bis pH 1 erreicht war. Durch Absaugen, Waschen mit Wasser und Trocknen wurden 580 g Feststoff erhalten. Dieses Rohprodukt wurde aus wässerigem Methanol umkristallisiert, wobei sich 435 g einer farblosen Mischung aus 90% N-Acetyl-L-leucin und 10% N-Acetyl-L-isoleucin ergaben.

86,6 g dieser Mischung wurden in 1 Liter 0,5 N Natronlauge gelöst und der pH-Wert wurde auf 7,4 eingestellt. Als Katalysator wurden 40 mg Nierenacylase aus Schweinenieren (1200 U/mg) und 60 mg $CoCl_2 \times 6\ H_2O$ zugegeben. Die klare Lösung wurde auf 39° C erwärmt. Nach 2¾ Stunden bildeten sich die ersten Kristalle. Nach 10 Stunden wurde auf 20° C abgekühlt und der voluminöse Niederschlag abgenutscht und mit Wasser gewaschen.

Nach dem Trocknen ergaben sich 35,6 g reines L-Leucin. Der Drehwert $[\alpha]_D^{25}$ betrug +15,3°.

Das Filtrat blieb 24 Stunden bei 20° C stehen, wobei sich erneut Kristalle abschieden. Nach Absaugen, Waschen mit Wasser und Trocknen ergaben sich weitere 9,3 g reines L-Leucin mit einem Drehwert $[\alpha]_D^{25} = +16{,}2°$.

*Beispiel 2:*

Eine aus der chromatographischen Trennung eines Proteinhydrolysates erhaltene Fraktion neutraler Aminosäuren hatte folgende Zusammensetzung:

| | |
|---|---|
| Leu | 49,8% |
| Ile | 36,1% |
| Val | 3,5% |
| Met | 3,5% |
| Tyr | 2,5% |
| Ala | 2,1% |
| Phe | 1,6% |
| Gly | 0,9% |

525 g dieser Mischung wurden in 2 Liter 2 N Natronlauge gelöst und auf 0° C gekühlt. Innerhalb 1 Stunde wurden 410 ml Acetanhydrid und gleichzeitig 850 ml 5 N Natronlauge eingerührt, wobei darauf geachtet wurde, dass der pH-Wert in einem Bereich zwischen pH 10 und 12 blieb und die Temperatur +5° C nicht überschritt. Nach beendeter Zugabe wurde noch 1 Stunde bei 5° C gerührt.

Die braune Lösung wurde mit konzentrierter Salzsäure verrührt, bis pH 1,5 erreicht war.

Durch Absaugen, Waschen mit Wasser und Trocknen wurden 530 g Acetylverbindungen als schwach braun gefärbtes Pulver erhalten. Dieses Rohprodukt wurde aus n-Butanol umkristallisiert, wobei sich 335 einer farblosen Mischung aus 65% N-Acetyl-L-leucin und 35% N-Acetyl-L-isoleucin ergaben.

86,6 g dieser Mischung wurden in 500 ml Wasser suspendiert und mit 50%iger Natronlauge verrührt, bis eine klare Lösung von pH 7,6 vorlag. Hierzu wurden 50 mg $CoCl_2 \times 6\ H_2O$ und 5 mg einer Aminoacylase aus Schweinenieren mit einer Aktivität von 2000 U/mg gegeben.

Die Lösung blieb 30 Stunden bei 38° C stehen. Danach wurde die entstandene Suspension auf 20° C abgekühlt und filtriert. Es ergaben sich 24,6 g reines L-Leucin mit einem Drehwert von $[\alpha]_D^{25} = +15{,}7°$.

## Patentansprüche

1. Verfahren zur Gewinnung von reinem L-Leucin aus Aminosäuregemischen, die mindestens 45 Gewichtsprozent L-Leucin, höchstens 40 Gewichtsprozent L-Isoleucin und höchstens 25 Gewichtsprozent an anderen Aminosäuren, jeweils bezogen auf Trockensubstanz, enthalten, dadurch gekennzeichnet, dass man

a) das Aminosäuregemisch in an sich bekannter Weise acetyliert,

b) aus dem rohen Gemisch der Acetylierungsprodukte durch Ansäuern mit einer Mineralsäure ein an N-Acetyl-L-leucin angereichertes Gemisch an N-Acetylaminosäuren ausfällt,

c) dieses an N-Acetyl-L-leucin angereicherte Gemisch in wässeriger Lösung mit einer Konzentration zwischen 0,1 und 1,5 Mol/l N-Acetyl-L-leucin bei einem pH zwischen 6 und 8 und einer Temperatur zwischen 10 und 40° C in Gegenwart eines Effektors einer Verseifung durch eine L-Aminosäureacylase unterwirft, bis 30 bis 95% des

eingesetzten N-Acetyl-L-leucins zur freien Aminosäure verseift sind, und

d) aus dem rohen Verseifungsgemisch das L-Leucin isoliert.

2. Verfahren nach Anspruch 1, dadurch gekennzeichnet, dass man das in der Verfahrensstufe b) ausgefällte Gemisch an N-Acetylaminosäuren vor Durchführung der Verfahrensstufe c) aus Wasser, einem mit Wasser mischbaren aliphatischen Alkohol mit 1 bis 4 Kohlenstoffatomen oder aus einem Gemisch von Wasser und einem solchen Alkohol umkristallisiert.

## Claims

1. A process for the recovery of pure L-leucine from amino acid mixtures which contain at least 45% by weight of L-leucine, at most 40% by weight of L-isoleucine and at most 25% by weight of other amino acids, based in each case on dry substance, characterised in that

a) the amino acid mixture is acetylated in known manner,

b) a mixture of N-acetyl amino acids enriched with N-acetyl-L-leucine is precipitated from the crude mixture of acetylation products by acidifying with a mineral acid,

c) this mixture enriched with N-acetyl-L-leucine is subjected to hydrolysis in an aqueous solution with a concentration of from 0.1 to 1.5 mol/l of N-acetyl-L-leucine by an L-amino acid acylase at a pH of from 5 to 8 and at a temperature of from 10 to 40° C in the presence of an activator, up to from 30 to 95% of the N-acetyl-L-leucine being hydrolysed to free amino acid, and

d) the L-leucine is isolated from the crude hydrolysis mixture.

2. A process according to claim 1, characterised in that the mixture of N-acetyl amino acids precipitated in the process stage b) is recrystallized before carrying out process stage c) from water, a water-miscible aliphatic alcohol having from 1 to 4 carbon atoms or a mixture of water and such an alcohol.

## Revendications

1. Procédé pour la préparation de L-leucine pure à partir de mélanges d'acides aminés qui contiennent au moins 45% en poids de L-leucine, 40% en poids au maximum de L-isoleucine et 25% en poids au maximum d'autres acides aminés, rapporté chaque fois à la substance sèche, caractérisé en ce que:

a) l'on effectue l'acétylation du mélange d'acides aminés d'une façon connue en soi,

b) l'on précipite, par acidification avec un acide minéral à partir du mélange brut des produits d'acétylation, un mélange d'acides N-acétylaminés enrichi en N-acétyl-L-leucine,

c) l'on soumet ce mélange enrichi en N-acétyl-L-leucine, en solution aqueuse, ayant une concentration en N-acétyl-L-leucine comprise entre 0,1 et 1,5 mole/l, à un pH entre 6 et 8 et à une température comprise entre 10 et 40° C, en présence d'un effecteur, à une saponification à l'aide d'une L-amino-acide-acylase, jusqu'à ce que 30 à 95% de la N-acétyl-L-leucine introduite soient saponifiés en acide aminé libre, et

d) l'on isole la L-leucine à partir du mélange de saponification brut.

2. Procédé selon la revendication 1, caractérisé en ce qu'avant d'effectuer le stade opératoire c, on recristallise dans de l'eau, dans un alcool aliphatique miscible à l'eau ayant de 1 à 4 atomes de carbone ou dans un mélange d'eau et d'un tel alcool, le mélange d'acides N-acétylaminés précipité dans le stade opératoire b.